# EUROPÄISCHE PATENTSCHRIFT

(11) **EP 1 190 676 B1**
(45) Veröffentlichungstag und Bekanntmachung des Hinweises auf die Patenterteilung: **13.08.2003**
(21) Anmeldenummer: 01102714.1
(22) Anmeldetag: 06.02.2001
(51) Int. Cl.: A61B 17/15

(54) **Vorrichtung zum Bestimmen der Position eines Schneidblocks**
Device for determining the position of a cutting guide
Dispositif pour déterminer la position d'un guide de coupe

(30) Priorität: 26.09.2000 EP 00120229
(43) Veröffentlichungstag der Anmeldung: 27.03.2002
(73) Patentinhaber: BrainLAB AG, 85551 Kirchheim/Heimstetten (DE)
(72) Erfinder: Neubauer, Timo, 80533 München (DE); Immerz, Martin, 81541 München (DE)
(74) Vertreter: Schwabe - Sandmair - Marx

(56) Entgegenhaltungen:
- WO-A-99/15097
- WO-A-99/60939
- DE-A- 19 639 615
- DE-U- 20 016 635
- DE-U- 29 704 393

## Beschreibung

Die vorliegende Erfindung bezieht sich auf ein System zur Positionierung eines Schneidblocks.

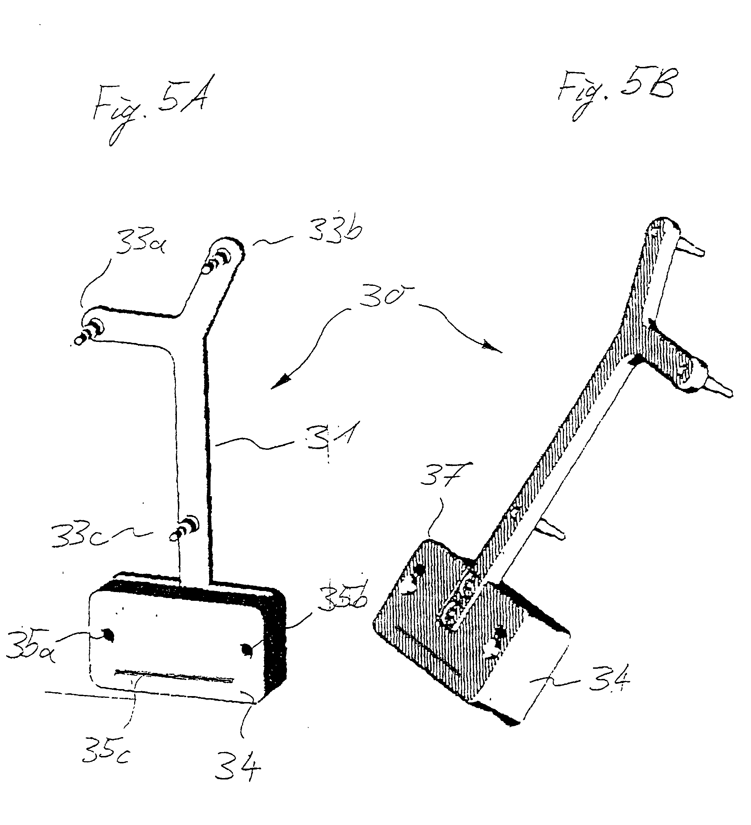

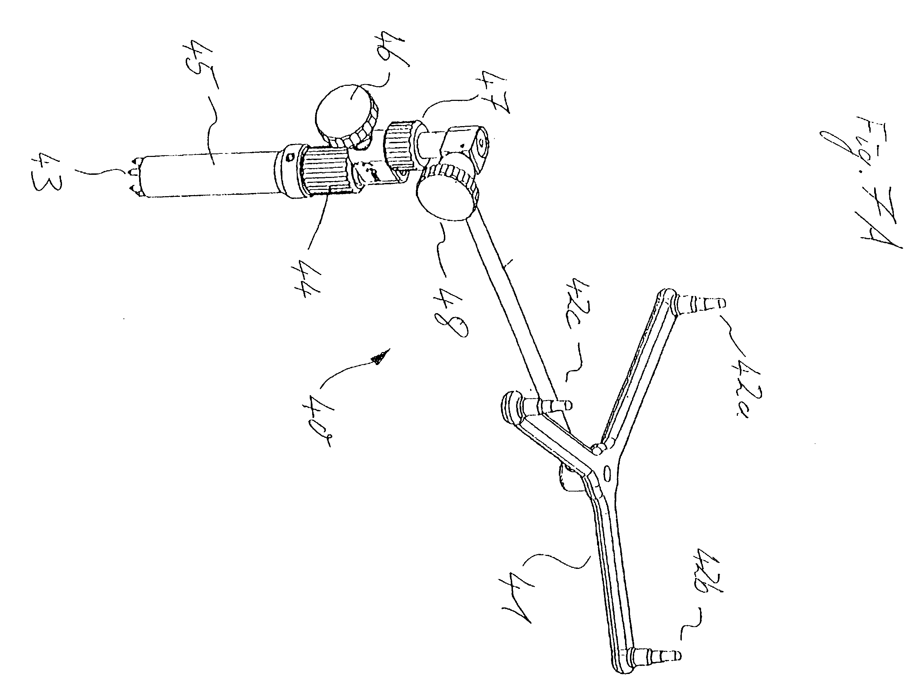

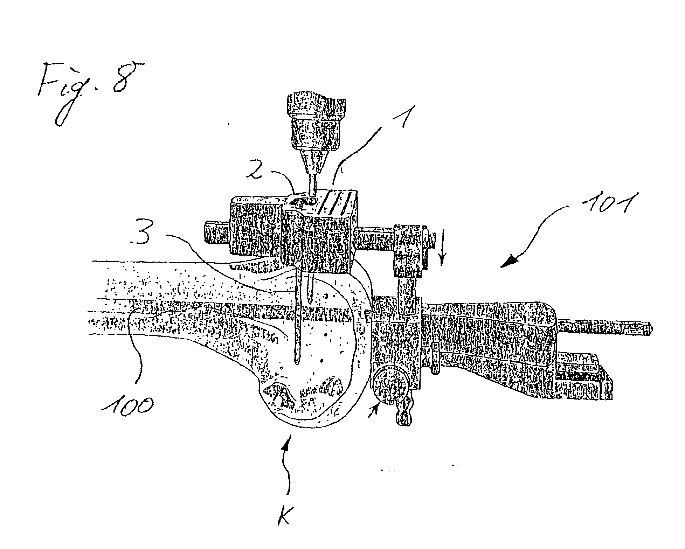

Beim Anbringen von Implantaten, wie zum Beispiel künstlichen Knie-, Ellbogen, Fingeroder Hüftgelenken, ist es erforderlich das Implantat, wie zum Beispiel ein Gelenk oder ein Knochenteil möglichst genau am angrenzenden Knochen zu positionieren. Hierzu müssen möglichst exakte Schnitte an den an das Gelenk angrenzenden Knochenstrukturen ausgeführt werden. Figur 8 zeigt schematisch die Positionierung eines Schneidblockes 1 an einem Oberschenkelknochen nach dem Stand der Technik. Hierzu wird eine Führungsstange 100 in einen Knochen K eingebracht, wobei am äußeren Ende der Führungsstange 100 eine Positionierungsmechanik 101 zum Positionieren und Halten des Schneidblockes 1 in einer gewünschten Position vorgesehen ist. Ist der Schneidblock 1 in die gewünschte Position gebracht worden, so können durch in dem Schneidblock 1 vorgesehene Löcher 2 Positionierungsstifte 3, beispielsweise Schrauben oder Nägel bzw. Pins in den Knochen K zur Fixierung des Schneidblocks 1 eingebracht werden. Nach erfolgter Fixierung des Schneidblockes 1 am Knochen K kann eine bevorzugt möglichst senkrecht oder leicht zur mechanischen Achse des Knochens abgewinkelt liegende erste Schnittebene S0 mittels eines Schneidwerkzeuges 4 erzeugt werden, wie schematisch in den Figuren 4A und 4B dargestellt. Um im Beispiel des Oberschenkelknochens die dazugehörige Knieimplantatkomponente auf dem Knochen positionieren zu können, müssen weitere Schnitte in weiteren zur Schnittebene S0 schräg verlaufenden oder abgewinkelten Ebenen S1 bis S4 erzeugt werden, wie in Figur 6 gezeigt. Hierzu wird ein zweiter Schneidblock 10 auf die erste Schnittebene S0 aufgesetzt und mittels einer geeigneten Mechanik positioniert, wobei mit diesem zweiten Schneidblock 10 weitere seitliche Schnitte am Knochen K erzeugt werden, welche durch Führung des Schneidwerkzeuges 4 in den schräg verlaufenden oder seitlichen Schlitzen des zweites Schneidblockes 10 erzeugt werden.

Aus der DE 297 04 393 U1 ist eine Vorrichtung zur präoperativen Bestimmung der Positionsdaten von Endoprothesenteilen eines mittleren Gelenks relativ zu den das mittlere Gelenk ausbildenden Knochen bekannt. Eine Sägeschablone trägt ein Markierungselement. Die ebene Führungsfläche der Sägeschablone wird durch ein geeichtes und handgeführtes Tastelement abgebildet, wobei ein solches Tastelement mit seiner Spitze an der Führungsfläche entlang geführt wird. Ein mit dem Tastinstrument verbundenes Markierungselement meldet dabei alle Positionsdaten des Tastelements zur Datenverarbeitungsanlage, die auf diese Weise Daten der Fläche aufnimmt, welche von der Spitze des Tastelementes abgefahren werden. Erst nach einem solchen Kalibrierungsvorgang stehen der Datenverarbeitungsanlage die Daten zur Verfügung, um aus der Orientierung des Markierungselementes die Orientierung der Führungsfläche zu berechnen.

Es ist die Aufgabe der vorliegenden Erfindung ein System zum vereinfachten Bestimmen der Position eines Schneidblockes vorzuschlagen.

Diese Aufgabe wird durch die Merkmale der unabhängigen Ansprüche gelöst. Vorteilhafte Ausführungsformen ergeben sich aus den Unteransprüchen.

Die Vorrichtung zum Bestimmen der Position eines Schneidblockes, insbesondere für ein chirurgisches Verfahren, wie zum Beispiel das Erzeugen einer oder mehrerer Schnittflächen für ein künstliches Gelenk, weist einen Referenzstern als Positionierungselement mit mindestens einem Referenzpunkt auf, dessen Position im Raum erfasst werden kann. Bei dem Positionierungselement kann es sich um aktive und/oder passive Elemente handeln, die geeignete Signale abgeben bzw. reflektieren, welche von entsprechenden Aufnahmegeräten, wie zum Beispiel Kameras, erfasst werden, um so die räumliche Lage des Positionierungselements und der damit verbundenen Vorrichtung bestimmen zu können. Weiterhin ist an der Vorrichtung ein Positionsbestimmungselement vorgesehen, durch welches die Position eines Schneidblocks an einem Knochen direkt oder indirekt festgelegt werden kann.

Ist auch die räumliche Lage des Knochens oder einer anderen Körperstruktur bekannt, so kann die relative Lage zum Beispiel zu dem Knochen bestimmt werden und basierend auf dieser Information eine Vorrichtung in eine gewünschte Position am Knochen gebracht werden. Allgemein wird dieser Vorgang als "Navigieren" bezeichnet.

Bei einer ersten Ausführungsform der Erfindung ist als Positionsbestimmungselement an der erfindungsgemäßen Vorrichtung ein Verbindungselement vorgesehen, welches in ein definiertes Lageverhältnis zu dem zu positionierenden Schneidblock gebracht und mit diesem verbunden bzw. daran befestigt werden kann. Dabei handelt es sich um ein oder mehrere plattenförmige Elemente, wobei mindestens eines dieser plattenförmigen Elemente in den Führungsschlitz eines Schneidblockes eingesteckt werden kann, um den Schneidblock in die gewünschte Position an einem Knochen zu bringen bzw. zu navigieren, in welcher bevorzugt nach Fixieren des Schneidblockes am Knochen und nach Entfernen der Vorrichtung ein Schnitt durch Führung eines geeigneten Schneidwerkzeuges durch den im Schneidblock befindlichen Führungsschlitz in der gewünschten Ebene erzeugt werden kann. Allgemein sind jedoch auch andere Verbindungen der Vorrichtungen mit dem Schneidblock möglich, so lange ein gewünschtes definiertes Lageverhältnis zwischen der Vorrichtung und dem Schneidblock eingehalten wird und es möglich ist die durch den Führungsscheitz definierte Schneidebene an die gewünschte Stelle zu navigieren.

Werden Platten oder in einer Ebene liegende Zapfen verwendet, welche in einen Fühnmgsschlitz des Schneidblockes eingesteckt werden können, so ist es vorteilhaft mehrere Platten oder Zapfen vorzusehen, welche bevorzugt in einer Ebene liegen und eine unterschiedliche Dicke oder Durchmesser aufweisen, um diese Platten oder Zapfen in Führungsschlitze unterschiedlicher Breite in den Schneidblock einstecken zu können.

Allgemein kann bei bekanntem Lageverhältnis zwischen Positionierungselement und Verbindungselement an der Vorrichtung das Verbindungselement zum Beispiel verschiebbar oder um eine Achse drehbar ausgestaltet sein. Werden zum Beispiel Platten zum Einstecken in Führungsschlitze eines Schneidblockes verwendet, so kann der Schneidblock auch bei Drehung oder eine Verschiebung der Platten in der gewünschten Ebene exakt positioniert werden, da die Drehung einer Platte um eine Achse parallel zur Normalen der Plattenoberfläche auch als Verschiebung in einer Ebene aufgefasst werden kann und der Schneidblock nur in Bezug auf die gewünschte theoretisch sich ins Unendliche erstreckende Schnittebene exakt positioniert werden soll. Eine Verschiebung des Schneidblockes bei welcher die durch den Führungsschlitz in dem Schneidblock definierte Schnittebene nicht verändert wird, ist somit unbeachtlich.

Gemäß der ersten Ausführungsform kann somit die Positionierung eines Schneidblockes durch das korrekte Ausrichten der gewünschten Schnittebene, also eine Navigation der Schnittebene bzw. des Führungsschlitzes des Schneidblockes erfolgen. Diese Art der Schneidblock-Positionierung wird bevorzugt zur Positionierung des ersten in Figur 4A und 4B gezeigten Scheidblockes zum Schneiden einer bevorzugt senkrecht auf oder leicht abgewinkelt zu der mechanischen Achse des Knochen stehenden Ebene verwendet.

Gemäß einer zweiten Ausführungsform der Erfindung, welche in Kombination mit der ersten Ausführungsform oder getrennt davon als unabhängige separate Ausführungsform verwendet werden kann, kann als Positionsbestimmungselement eine Vorrichtung zur Vorbereitung und/oder Erzeugung einer Verbindungsstruktur zwischen Knochen und Schneidblock verwendet werden.

Diese Vorrichtung zur Vorbereitung oder Erzeugung einer Verbindungsstruktur ist eine Lochlehre und/oder eine Spaltlehre mit mindestens einem durch die Lehre definierten durchgehendem Loch oder Spalt, welche unter Verwendung des mit der Lehre verbundenen Positionierungselementes in eine gewünschte Position zum Beispiel an einem Knochen gebracht wird. In der Lochlehre sind Führungslöcher vorgesehen, welche zum Bohren von Löchern in den Knochen oder zum Einstecken geeigneter Verbindungselemente, zum Beispiel Stifte, Nägel oder Schrauben in den Knochen dienen können. Bei einer Spaltlehre sind Führungsspalte vorgesehen, welche zum Sägen oder Fräsen von Nuten oder Spalten in den Knochen oder zum Einstecken geeigneter Verbindungselemente, zum Beispiel Platten in den Knochen dienen können. Möglich ist auch eine Kombination aus Loch- und Spaltlehre. An diese mit Hilfe der Lehre vorbereiteten Verbindungsstrukturen bzw. ausgerichteten Verbindungselemente, wie zum Beispiel mit der Lochlehre gebohrten Löcher kann ein zweiter Schneidblock angebracht bzw. aufgesteckt werden, wie zum Beispiel der in Figur 6 gezeigte zweite Schneidblock. Die Position des zweiten Schneidblocks wird somit anders als bei der ersten Ausführungsform nur indirekt durch die Lehre, zum Beispiel die Lochlehre bestimmt. Allgemein kann jedoch eine direkte oder indirekte Positionierung bei dem ersten oder zweiten Schneidblock erfolgen.

Dabei kann die Lehre zum Beispiel auf der mit Hilfe des ersten Schneidblockes erzeugten ersten Ebene S0 verschoben werden und muss lediglich in eine gewünschte Position auf dieser Ebene S0 verschoben werden, wodurch keine vollständige Navigation im dreidimensionalen Raum erforderlich ist. Jedoch kann auch eine solche dreidimensionale Navigation der Lehre zu der gewünschten Stelle vorgenommen werden.

Zur Fixierung der Lehre auf der bereits erzeugten Ebene S0 können auf der Auflagefläche der Lehre Fixierungselemente, zum Beispiel Spikes dienen, die sich durch leichten Druck auf der gegenüberliegenden Seite der Lehre in die weiche Struktur des Knochengewebes eindrücken lassen.

Als Positionierungselement ist ein Referenzstern mit bevorzugt drei darauf angeordneten passiven Markern vorgesehen, welche zur Navigation der ersten Ausführungsform durch Einstecken eines Plättchens in einen Führungsschlitz der Schnittlehre bevorzugt eine erste Geometrie aufweisen, welche von der Geometrie bzw. Anordnung zur Navigation der Lochlehre der zweiten Ausführungsform bevorzugt verschieden ist. Hierdurch kann eine eindeutige Zuordnung der Referenzsterne zu den jeweils durch diese Referenzsterne navigierten Elementen vorgenommen werden.

Das erfindungsgemäße System zum Bestimmen der Position eines Schneidblockes besteht aus der oben beschriebenen Vorrichtung und dem Schneidblock selbst, wie bei der ersten Ausführungsform beschrieben, oder der oben beschriebenen Vorrichtung und einer Vorrichtung zur Vorbereitung und/oder Erzeugung einer Verbindungsstruktur, d.h. der Lochlehre, welche bei der zweiten Ausführungsform beschrieben wurde.

Bevorzugt sind an der zu bearbeitenden Körperstruktur, zum Beispiel einem zu schneidenden Knochen ebenfalls geeignete Positionierungselemente, wie zum Beispiel ein Referenzstern, vorgesehen, um aus der hierdurch gewonnenen räumlichen Lageinformation in Verbindung mit der Lageinformation des geführten Schneidblocks oder der Lochlehre eine exakte Navigation dieser Vorrichtungen durchführen zu können.

Bei einem mit dem erfindungsgemäßen System durchgeführten Verfahren zum Bestimmen der Position oder zum Navigieren eines Schneidblockes wird zunächst die Position der an einer Körperstruktur, zum Beispiel an einem Knochen angebrachten Positionierungselemente bestimmt, aus welchen die räumliche Lage zum Beispiel des Knochens oder eines Gelenks ermittelt werden kann. Weiterhin wird die räumliche Lage der Positionierungselemente eines Schneidblockes oder einer Lochlehre bestimmt, so dass aus diesen beiden Positionsinformationen das relative Lageverhältnis zwischen Körperstruktur, zum Beispiel Knochen und Schneidblock oder Lochlehre ermittelt werden kann. Basierend auf dieser relativen Position kann nun eine Navigation des Schneidblockes bzw. der Lochlehre zu einer gewünschten Stelle erfolgen. Hierzu können geeignete Anzeigevorrichtungen vorgesehen sein, über welche das relative Lageverhältnis dargestellt wird.

Die Erfindung wird nachfolgend anhand bevorzugter Ausführungsformen beschrieben werden. Es zeigen:
- Figur 1: eine Vorrichtung zur Positionierung eines Schneidblocks gemäß der ersten Ausführungsform;
- Figuren 2A bis 2D: verschiedene Ansichten eines tibialen Schneidblocks;
- Figur 3: zwei Ansichten eines femoralen Schneidblocks;
- Figuren 4A und 4B: das Schneiden eines Knochens mit einem gemäß der ersten Ausführungsform positionierten ersten Schneidblock;
- Figuren 5A und 5B: eine navigierbare Spalt- und Lochlehre gemäß der zweiten Ausführungsform;
- Figur 6: das Schneiden weiterer Schnittebenen S1 bis S4 mit einem gemäß der zweiten Ausführungsform positionierten zweiten Schneidblock;
- Figuren 7A und 7B: zwei Ausführungsformen einer zum Anbringen eines Referenzsternes an einem Knochen geeigneten erfindungsgemäßen Klemmvorrichtung;
- Figur 8: die Positionierung eines Schneidblockes nach dem Stand der Technik.

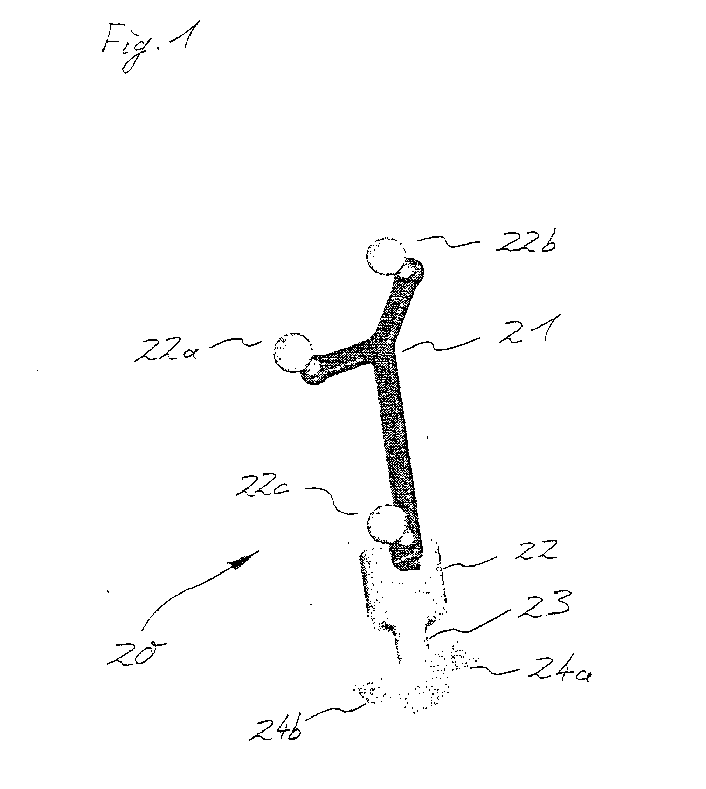

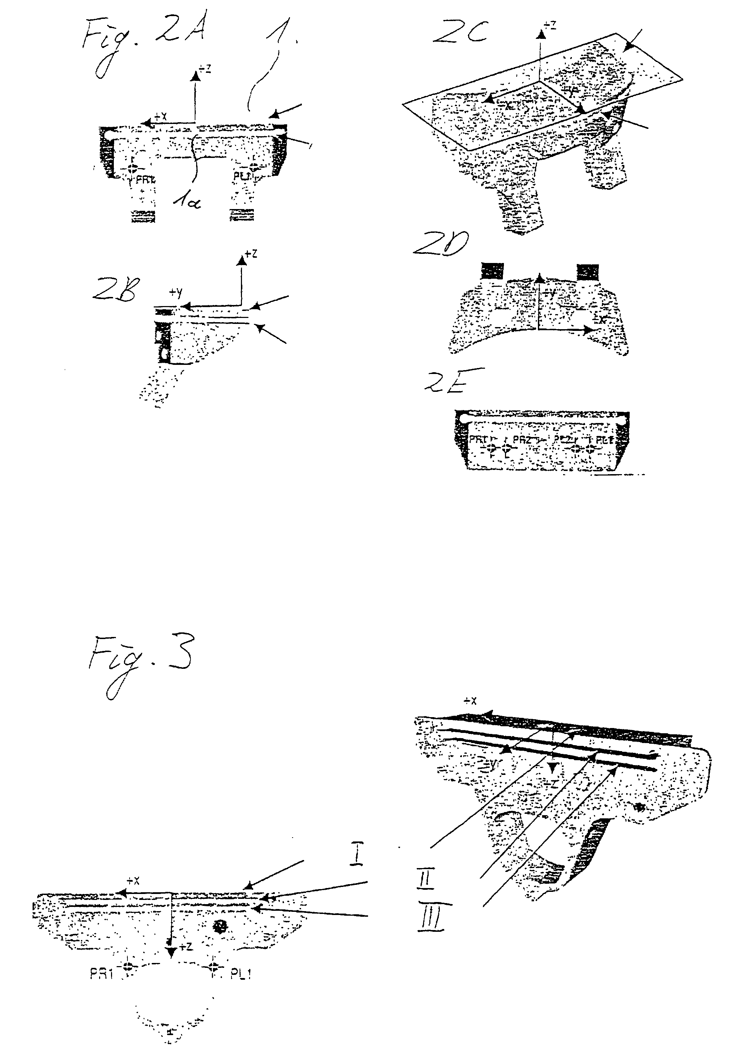

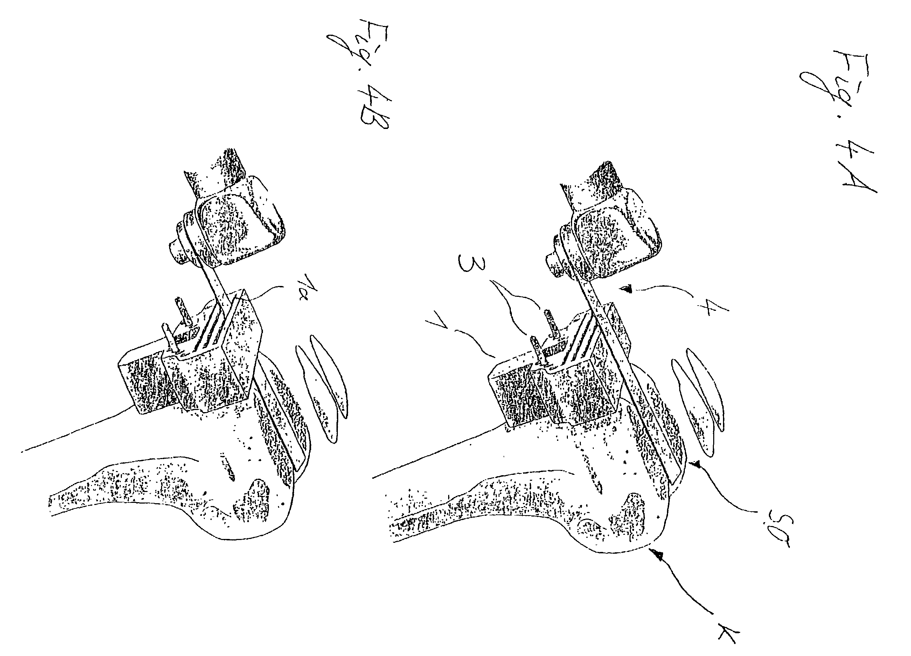

Figur 1 zeigt eine erste Ausführungsform der Vorrichtung 20 mit einem Referenzstern 21 und darauf angeordneten kugelförmigen Elementen 22a bis 22c mit reflektierender Oberfläche. Zwei Infrarotkameras (nicht gezeigt) erfassen ein an den kugelförmigen Elementen 22a bis 22c, welche auch als Marker bezeichnet werden, reflektiertes Licht und kann hieraus die Lage des Referenzsternes 21 im Raum ermitteln. Der Referenzstern 21 ist fest mit einem Grundkörper 22 verbunden, an welchem ein drehbares Element 23 angebracht ist. Die Achsrichtung des Drehelementes kann in der gezeigten Ausführungsform in seiner festgelegten Orientierung zum Referenzstem 21 nicht verändert werden. Am äußeren Ende des drehbaren Elementes 23 sind zwei Platten 24a, 24b unterschiedlicher Dicke angeordnet, welche so ausgerichtet sind, dass die Platten, 24a, 24b in einer Ebene liegen und die Normale auf die jeweiligen Plattenebenen parallel zur Drehebene des drehbaren Elementes 23 ist. Eine dieser Platten 24a, 24b kann in einen Führungsschlitz 1a des in den Figuren 2A bis 2E gezeigten tibialen Scheidblockes oder einen der Führungsschlitze II, III des in Figur 3 gezeigten femoralen Schneidblockes eingesteckt werden. Dabei ist zum Erzeugen des in Figur 4 gezeigten Schnittes in einer ersten Schnittebene S0 lediglich das Navigieren des Schneidblockes 1 bezüglich der gewünschten Schnittebene, d. h. das auf den Führungsschlitz 1a bezogene Navigieren erforderlich. Hierbei kann der Führungsschlitz 1a in der gewünschten Schnittebene verschoben werden, da die durch den Schneidblock 1 geführte Schneidvorrichtung 4 nur in der gewünschten Schnittebene S0 geführt werden muss.

Figur 3 zeigt einen femoralen Schneidblock, wobei eine Führung eines Schneidgerätes entweder durch die in Figur 3 oben gezeigte Auflagefläche I oder durch eine der beiden Führungsschlitze II oder III erfolgen kann.

Wird zum Beispiel der in Figur 2 gezeigte tibiale Schneidblock 1 mit dem in den Führungsschlitz 1a eingesteckten in Figur 1 gezeigten Positionierungselement 20 an einen Knochen K navigiert, so kann dieser Schneidblock 1 mit geeigneten Befestigungs- oder Halteelementen 3 am Knochen K befestigt werden, wie in Figur 4A gezeigt. Mit einem Schneidwerkzeug 4 kann ein gewünschter Schnitt in der Schnittebene S0 entweder durch Auflegen einer Klinge auf die obere Seite des Schneidblockes 1, oder durch Führen einer Klinge in einem Führungsschlitz 1a, wie in Figur 4B gezeigt, erfolgen.

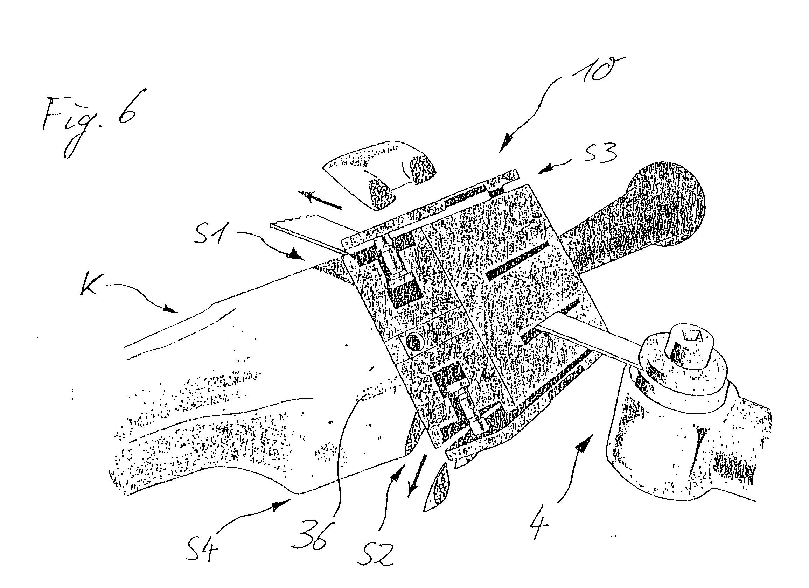

Die in den Figuren 5A und 5B gezeigte zweite Ausführungsform zur Bestimmung der Position eines Schneidblockes weist ebenso wie die in Figur 1 gezeigte erste Ausführungsform einen Referenzstem 31 auf, wobei in den Figuren 5A und 5B nur die Halteelemente 33a bis 33c zum Aufstecken von kugelförmigen Elementen mit reflektierenden Oberflächen gezeigt sind. Mit dem Referenzstem 31 ist eine Bohrlehre 34 mit zwei darin vorgesehenen Bohrlöchern 35a, 35b fest verbunden. An der in Figur 5B gezeigten Unterseite der Lehre 34 können Spikes 37 zum vorläufigen Fixieren der Lehre 34 vorgesehen sein. Die in Figur 5 gezeigte Bohrlehre kann durch den Referenzstern 31 so auf der in Figur 4 gezeigten Schnittebene S0 positioniert werden, dass durch die Löcher 35a und 35b geeignete Werkzeuge zur Erzeugung einer Verbindungsstruktur zwischen Knochen und Schneidblock, zum Beispiel Halteelemente 36 in den Knochen K eingeschoben werden, so dass der zweite Schneidblock 10, wie schematisch in Figur 6 gezeigt, durch das Aufstecken auf das bzw. die Halteelemente 36 richtig positioniert werden kann. Ebenso kann zum Beispiel auch ein Halteelement 36 fest mit dem Schneidblock 10 verbunden sein, so dass der Schneidblock 10 mit Halteelement 36 in die mit Hilfe der Bohrlehre 34 in den Knochen K gebohrten Löcher eingesteckt werden kann. Ist der zweite Schneidblock 10 in die gewünschte Position gebracht worden, so können durch die verschiedenen in dem zweiten Schneidblock 10 vorgesehenen Führungsschlitze die gewünschten Schnitte in den Ebenen S1 bis S4 ausgeführt werden.

Auf einen Knochen, bei welchem mit Hilfe des ersten Schneidblockes 1 und des zweiten Schneidblockes 10 die gewünschten Schnittebenen S0 bis S4 erzeugt wurden, kann ein künstliches Gelenk aufgesetzt werden, welches bei richtiger Lage der Schnittebenen S0 bis S4 auch richtig positioniert ist.

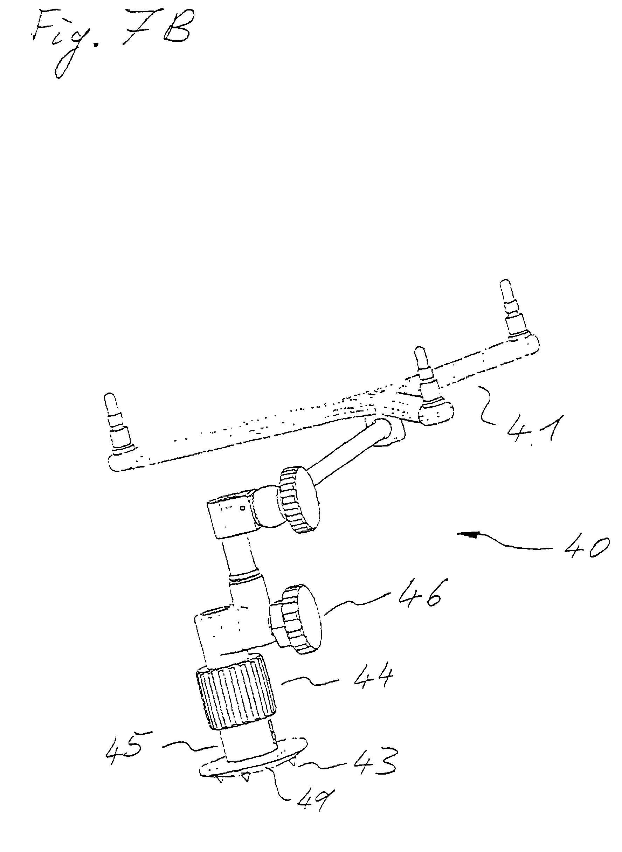

Figur 7A zeigt eine erste Vorrichtung 40, mit welcher ein Referenzstern 41 mit darauf angebrachten Halterungen 42a bis 42c zum Aufstecken von nicht gezeigten reflektierenden Markern in einem definierten Lageverhältnis an einem Knochen oder einer anderen Struktur befestigt werden kann. Der zum Beispiel an einem Knochen befestigte Referenzstern 41 dient als Bezugssystem zur Navigation und Positionierung der in Figur 1 und Figur 5 gezeigten Vorrichtungen. Jedoch kann die in Figur 7 gezeigte Vorrichtung auch unabhängig von den oben beschriebenen Elementen und Vorrichtungen verwendet werden.

Die an der Unterseite der Vorrichtung 40 gezeigten Spikes 43, welche mit der Hülse 45 verbunden sind, können durch ein Drehen der Schraube 44 ein- und ausgefahren werden. Wird zum Beispiel eine Stange, wie zum Beispiel eine Schantzschraube oder ein Kirschnerdraht, in einen Knochen zum Anbringen der Vorrichtung 40 eingebracht, so kann die Vorrichtung 40 mit der Hülse 45 auf diese im Knochen angebrachte Stange aufgesteckt werden. Die Schraube 46 dient zur Fixierung der Vorrichtung 40 an dieser Stange. Nach erfolgter Fixierung mittels der Schraube 46 kann die Mutter 44 gedreht werden, um die Spikes 43 nach unten zu bewegen und so ein Verspannen der Vorrichtung 40 bezüglich des Knochens oder eines anderen Elementes, wie zum Beispiel Haut oder auf Haut aufgebrachte Elemente zu bewirken. Es kann somit eine ortsfeste nicht verschiebbare Verbindung eines Referenzsterns 41 an nur einer einzelnen Stange in einem Knochen erhalten werden, da eine Verspannung über die ein- bzw. ausfahrbaren Spikes 43 möglich ist. Die Position des Referenzsterns 41 kann mittels der Schrauben 47 und 48 durch Drehung um zwei Achsen verändert werden.

Figur 7B zeigt eine zweite Ausführungsform einer Vorrichtung 40 zum ortsfesten Anbringen eines Referenzsternes 41, wobei die Spikes 43 auf einer Abstützfläche 49 angeordnet sind und mit der Fläche 49 nach unten bewegt werden, um die Vorrichtung 40 so an der Hautoberfläche fest zu verspannen.

Obwohl die Erfindung insbesondere im Hinblick auf Knie-Implantate beschrieben wurde kann diese natürlich auch bei anderen Implantaten oder Verfahren verwendet werden. Dabei können insbesondere die Vorrichtung zur Navigation des Schneidblocks, die Vorrichtung zur Navigation der Lochlehre und die in Figur 7 gezeigte Klemmvorrichtung unabhängig voneinander verwendet werden.

## Patentansprüche

1. System zum Positionieren eines Schneidblocks (1) mit:
- einem Referenzstern (21) mit mindestens einem Referenzpunkt (22a, 22b, 22c) dessen räumliche Position erfasst werden kann;
- einem Schneidblock (1) mit einem Führungsschlitz (la); und
- mindestens einer in den Führungsschlitz (la) des Schneidblocks (1) einsteckbaren Platte (24a, 24b), welche fest mit dem Referenzstern (21) verbunden ist.

2. System nach Anspruch 1, wobei mindestens zwei Platten (24a, 24b) vorgesehen sind, welche bevorzugt eine unterschiedliche Dicke aufweisen.

3. System nach Anspruch 1 oder 2, wobei mindestens eine Platte (24a, 24b) um eine Achse parallel zu ihrer Normalen drehbar und/oder verschiebbar ist.

4. System zum Positionieren eines Schneidblocks (10) mit:
- einem Referenzstern (31) mit mindestens einem Referenzpunkt (33a, 33b, 33c), dessen räumliche Position erfasst werden kann;
- einem Schneidblock (10) mit Löchern; und
- einer Lochlehre (34), welche fest mit dem Referenzstern (31) verbunden ist und Löcher (35a, 35b) aufweist, welche korrespondierend zu Löchern des Schneidblocks (10) angeordnet sind.

5. System zum Positionieren eines Schneidblocks (10) mit:
- einem Referenzstern (31) mit mindestens einem Referenzpunkt (33a, 33b, 33c), dessen räumliche Position erfasst werden kann;
- Halteelementen (36);
- einem Schneidblock (10), welcher auf die Halteelemente (36) aufgesteckt werden kann; und
- einer Lochlehre (34), welche fest mit dem Referenzstern (31) verbunden ist und Löcher (35a, 35b) zum Einschieben der Halteelemente (36) aufweist.

6. System nach Anspruch 5, wobei die Lochlehre (34) mindestens zwei Löcher (35a, 35b) aufweist.

7. System nach einem der vorhergehenden Ansprüche, wobei der Referenzstern (21, 31) mindestens drei Referenzpunkte (22a, 22b, 22c; 33a, 33b, 33c) mit reflektierender Oberfläche aufweist.

8. System nach einem der vorhergehenden Ansprüche mit einer Vorrichtung (40) zum lagefesten Anbringen eines Positionierungselementes (41) an einem Knochen.

## Claims

1. A system for positioning an incision block (1), comprising:
- a reference star (21), with at least one reference point (22a, 22b, 22c), whose spatial position can be detected;
- an incision block (1), with a guide slit (1a); and
- at least one plate (24a, 24b) which may be inserted into the guide slit (1a) of the incision block (1) and which is firmly connected to said reference star (21).

2. The system as set forth in claim 1, wherein at least two plates (24a, 24b) are provided, which are preferably of different thicknesses.

3. The system as set forth in claim 1 or 2, wherein at least one plate (24a, 24b) can be rotated and/or shifted about an axis, parallel to its vertical.

4. A system for positioning an incision block (10), comprising:
- a reference star (31), with at least one reference point (33a, 33b, 33c), whose spatial position can be detected;
- an incision block (10), with holes; and
- a hole template (34) which is firmly connected to said reference star (31) and comprises holes (35a, 35b) which are arranged in correspondence with holes of the incision block (10).

5. A system for positioning an incision block (10), comprising:
- a reference star (31), with at least one reference point (33a, 33b, 33c), whose spatial position can be detected;
- holding elements (36);
- an incision block (10), which can be slipped onto said holding elements (36); and
- a hole template (34) which is firmly connected to the reference star (31) and comprises holes (35a, 35b) for inserting the holding elements (36).

6. The system as set forth in claim 5, wherein said hole template (34) comprises at least two holes (35a, 35b).

7. The system as set forth in any one of the preceding claims, wherein the reference star (21, 31) comprises at least three elements (22a, 22b, 22c; 33a, 33b, 33c) with reflecting surfaces.

8. The system as set forth in any one of the preceding claims, comprising a device (40) for attaching a positioning element (41) to a bone, in a stable manner.

## Revendications

1. Système de positionnement d'un guide de coupe (1), qui comporte :
- une étoile de référence (21) qui présente au moins un point de référence (22a, 22b, 22c) dont la position dans l'espace peut être détectée,
- un guide de coupe (1) qui présente une fente de guidage (la) et
- au moins une plaque (24a, 24b) qui peut être insérée dans la fente de guidage (la) du guide de coupe (1) et qui est reliée rigidement à l'étoile de référence (21).

2. Système selon la revendication 1, dans lequel sont prévues au moins deux plaques (24a, 24b) qui présentent de préférence des épaisseurs différentes.

3. Système selon la revendication 1 ou 2, dans lequel au moins une plaque (24a, 24b) peut tourner autour d'un axe parallèle à sa normale et/ou peut coulisser.

4. Système de positionnement d'un guide de coupe (10), qui comporte :
- une étoile de référence (31) qui présente au moins un point de référence (33a, 33b, 33c) dont la position dans l'espace peut être détectée,
- un guide de coupe (10) qui présente des perforations et
- un gabarit perforé (34) qui est relié rigidement à l'étoile de référence (31) et qui présente des perforations (35a, 35b) qui sont disposées en correspondance à des perforations du guide de coupe (10).

5. Système de positionnement d'un guide de coupe (10), qui comporte :
- une étoile de référence (31) qui présente au moins un point de référence (33a, 33b, 33c) dont la position dans l'espace peut être détectée,
- des éléments de maintien (36),
- un guide de coupe (10) qui peut être enfiché sur les éléments de maintien (36) et
- un gabarit perforé (34) qui est relié rigidement à l'étoile de référence (31) et qui présente des perforations (35a, 35b) pour l'insertion des éléments de maintien (36).

6. Système selon la revendication 5, dans lequel le gabarit perforé (34) présente au moins deux perforations (35a, 35b).

7. Système selon l'une des revendications précédentes, dans lequel l'étoile de référence (21, 31) présente au moins trois points de référence (22a, 22b, 22c; 33a, 33b, 33c) à surface réfléchissante.

8. Système selon l'une des revendications précédentes, qui comporte un dispositif (40) d'installation en position fixe d'un élément de positionnement (41) sur un os.
